(19)

**Europäisches Patentamt**

**European Patent Office**

**Office européen des brevets**

(11) **EP 4 675 640 A1**

(12) **EUROPEAN PATENT APPLICATION**

(43) Date of publication:
**07.01.2026 Bulletin 2026/02**

(21) Application number: **24186862.9**

(22) Date of filing: **05.07.2024**

(51) International Patent Classification (IPC):
**G16H 50/20** (2018.01)     **G16H 50/30** (2018.01)
**G16H 50/70** (2018.01)

(52) Cooperative Patent Classification (CPC):
**G16H 50/30; G16H 20/40; G16H 50/20;
G16H 50/70**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **X-cardiac GmbH
10707 Berlin (DE)**

(72) Inventors:
• **MEYER, Alexander
10707 Berlin (DE)**
• **BROSIEN, Kay
10707 Berlin (DE)**
• **HOPPE, Florian
10707 Berlin (DE)**
• **HELLMEIER, Florian
10707 Berlin (DE)**

(74) Representative: **Pfenning, Meinig & Partner mbB
Patent- und Rechtsanwälte
Joachimsthaler Straße 10-12
10719 Berlin (DE)**

(54) **MEDICAL DATA PROCESSING DEVICE, METHOD FOR GENERATING A PREDICTION FUNCTION, METHOD FOR DETERMINING A RISK INDICATOR, DATA PROCESSING SYSTEM, AND COMPUT-ER PROGRAM PRODUCT**

(57)     The disclosure concerns, inter alia, a medical data processing device (100, 110, 120), configured to receive or retrieve a pre-generated prediction function, the prediction function configured to determine a risk indicator (800) for a medical complication event associated with a patient during a hospitalization in an intensive care unit and/or an intermediate care unit and/or a telemetry unit of a first clinical site (601), in particular following a surgery, the pre-generated prediction function generated by training a machine learning model (701) using external training data (210) comprising patient data originating from one or more second clinical sites (602), the patient data comprising manually and/or automatically collected data of a set of measurement-based clinical parameters associated with a plurality of patients, the data comprising at least a subset of the clinical parameters collected with a plurality of different respective sampling frequencies; and assess (S420) the performance of the prediction function (S420) by generating at least one performance metric by using, as the input of the prediction function, local assessment data (320) comprising patient data originating from the first clinical site (601).

FIG. 6

**EP 4 675 640 A1**

**Description**

The disclosure concerns a method for generating a prediction function, the prediction function configured to determine a risk indicator for a medical complication event, a method for determining such a risk indicator, as well as a corresponding data processing system, medical data processing device, and computer program product. The disclosure relates to the field of clinical data processing and is directed at generating predictors of clinical events and assessing the performance of such predictors, in particular based on time series of measured patient data used as training and assessment data. The subject matter of the disclosure is applicable, for instance, in postsurgical patient care such as during hospitalization in an intensive care unit and/or an intermediate care unit and/or a telemetry unit.

In a medical or clinical context, in particular in settings such as an intensive care unit and/or an intermediate care unit and/or a telemetry unit, healthcare personnel are often presented with a large and complex array of patient-associated data, including measurement-based (i.e. measured and/or measurement-derived) values of numerous time-varying clinical parameters (in particular, time series of both current and previous values). Such data may inform not only on the existence of current health-related conditions, but also on the risk of future health-related events. Such events include complications associated with existing conditions or completed or ongoing medical - for instance, surgical - treatments or interventions.

The use of predictive computational models, for instance machine learning models, for forecasting the risk of future events of these types based on such data in order to support medical assessment and decision-making by healthcare personnel has been proposed. However, the design, implementation, and deployment of such models face numerous challenges. For instance, the present inventors have recognized that model performance may be negatively affected both by inhomogeneities within a given dataset (such as different sampling frequencies or measurement equipment) and by various types of dataset shift, i.e. systematic differences between the data used to generate a model and/or assess its performance and the data used as an input of the deployed model. Such dataset shift may be caused, for instance, by differences in the composition of patient cohorts (such as demographics or disease spectrum), differences in the clinical environment (such as equipment, procedures, or personnel expertise level), and/or changes of any such aspects over time.

Correspondingly, the present disclosure aims to provide solutions or improvements regarding at least some of the aforementioned aspects. To this end, the subject matter of the independent claims is provided. Further advantageous aspects and optional features are disclosed in the dependent claims and in the description.

A method is provided for generating a prediction function, the prediction function configured to determine a risk indicator for a medical complication event associated with a patient during a hospitalization in an intensive care unit and/or an intermediate care unit and/or a telemetry unit of a first clinical site the method comprising:

initially generating the prediction function by training a machine learning model using external training data comprising patient data originating from one or more second clinical sites, the patient data comprising manually and/or automatically collected data of a set of measurement-based clinical parameters associated with a plurality of patients, the data comprising at least a subset of the clinical parameters collected with a plurality of different respective sampling frequencies;

initially assessing a performance of the prediction function by generating at least one performance metric by using, as the input of the prediction function, external assessment data comprising patient data originating from the one or more second clinical sites; and

additionally assessing the performance of the prediction function by generating at least one performance metric by using, as the input of the prediction function, local assessment data comprising patient data originating from the first clinical site.

Using a machine learning model (such as a deep-learning neural network) as a basis for the prediction function as described is intrinsically capable of accounting for inhomogeneities within the external training data, including the differences in respective sampling frequencies of different clinical parameters comprised in the corresponding patient data, and thus reducing or avoiding negative impacts of such inhomogeneities on model and prediction function performance. The combination of initial assessment (based on external assessment data) and additional assessment (based on local assessment data) enables monitoring of negative impacts of any dataset shift of the aforementioned types on model and prediction function performance, which, in turn, enables reduction or avoidance of such impacts by, for instance, updating the prediction function and/or replacement of the prediction function by a more appropriate one in order to account for the dataset shift.

A method for determining a risk indicator of the aforementioned type is further provided, the method comprising:

acquiring, during the hospitalization, manually and/or automatically collected clinical data of the set of measurement-based clinical parameters associated with the patient, the data comprising at least a subset of the clinical parameters collected with a plurality of different respective sampling frequencies; and

determining the risk indicator by using a prediction function generated using the method for generating a prediction function described above, wherein the prediction function takes the clinical data as an input.

According to the foregoing, determination of a risk indicator in the described manner enables reduction or avoidance of negative impacts of data inhomogeneities and/or dataset shift on model and prediction function performance. The risk indicator may be used, by healthcare personnel, as a factor - in particular as one of a plurality of pertinent factors - involved in medical decision making, for example in the determination of whether further medical interventions are indicated or whether a particular medical condition could be present or expected. It is further noted that the use of a machine learning model as described enables the determination of a risk indicator for a potential future medical complication event merely based on the identification of certain patterns within the clinical data used as an input without the need to identify the specific presence or nature of any underlying condition or pathology.

The machine learning model is preferably a neural network. A neural network comprises a plurality of layers, wherein each layer comprises a plurality of nodes; connections between the nodes are associated with respective weights. The machine learning model may be trained by supervised learning, though un-supervised, semi-supervised and/or self-supervised approaches are also conceivable. The machine learning model preferably is or comprises a deep learning model. A transformer model, for instance, has been proven to exhibit particularly favorable performance in the context of the present disclosure. Other types of models, such as a recurrent neural network (RNN), logistic regression, support vector machine, or random forest may alternatively be used.

A source of information regarding the principles and implementation of transformer models is Zerveas et al., A Transformer-based Framework for Multivariate Time Series Representation Learning, arXiv:2010.02803v3 [cs.LG] (2020), doi:10.48550/arXiv.2010.02803, https://arxiv.org/abs/2010.02803v3. Information regarding different machine learning models with further references may be found, for instance, in Krittanawong et al., Artificial Intelligence in Precision Cardiovascular Medicine, Journal of the American College of Cardiology, Volume 69, Issue 21 (2017), Pages 2657-2664, ISSN 0735-1097, doi:10.1016/j.jacc.2017.03.571.

A medical data processing device is further disclosed, the device configured to

receive or retrieve a pre-generated prediction function, the prediction function configured to determine a risk indicator for a medical complication event associated with a patient during a hospitalization in an intensive care unit and/or an intermediate care unit and/or a telemetry unit of a first clinical site, in particular following a surgery, the pre-generated prediction function generated by training a machine learning model using external training data comprising patient data originating from one or more second clinical sites, the patient data comprising manually and/or automatically collected data of a set of measurement-based clinical parameters associated with a plurality of patients, the data comprising at least a subset of the clinical parameters collected with a plurality of different respective sampling frequencies; and

assess the performance of the prediction function by generating at least one performance metric by using, as the input of the prediction function, local assessment data comprising patient data originating from the first clinical site.

The medical data processing device may further be configured to

acquire, during the hospitalization, manually and/or automatically collected clinical data of the set of measurement-based clinical parameters associated with the patient, the data comprising at least a subset of the clinical parameters collected with a plurality of different respective sampling frequencies;

determine the risk indicator by using the prediction function, wherein the prediction function takes the clinical data as an input.

The disclosure further provides a data processing system (i.e. a system of one or several data processing devices) comprising means for executing any of the above-described methods (i.e. either or both of generating the prediction function and determining the risk indicator) as well as a computer program product comprising instructions which, when executed on a data processing device, cause the data processing device to execute any of the above-described methods.

The medical data processing device and/or the data processing system and/or the computer program product may further comprise optional features corresponding to any of the optional method steps or aspects provided in this disclosure; conversely, the proposed methods may comprise method steps or aspects corresponding to the use of any of the features disclosed in conjunction with the medical data processing device and/or the data processing system and/or the computer program product.

It will be appreciated that the medical data processing device and/or the data processing system may be implemented, for instance, using general-purpose and/or dedicated hardware, local and/or distributed computing architectures and the like. The instructions for execution of the disclosed methods may be retrievable, for instance, from an internal and/or external storage medium and/or from a server via a network connection. The provided functionality as a whole or individual aspects thereof may be implemented in hardware and/or softwa re.

The medical data processing device and/or the data processing system may be or comprise a prediction function generation unit for generating a prediction function, the prediction function configured to determine a risk indicator for a medical complication event associated with a patient during a hospitalization in an intensive care unit and/or an intermediate care unit and/or a telemetry unit of a first clinical site, in particular following a surgery, the prediction function generation unit configured to

> initially generate the prediction function by training a machine learning model using external training data comprising patient data originating from one or more second clinical sites, the patient data comprising manually and/or automatically collected data of a set of measurement-based clinical parameters associated with a plurality of patients, the data comprising at least a subset of the clinical parameters collected with a plurality of different respective sampling frequencies;
> initially assess a performance of the prediction function by generating at least one performance metric by using, as the input of the prediction function, external assessment data comprising patient data originating from the one or more second clinical sites.

The medical data processing device and/or the data processing system may be or comprise a risk indicator determination unit for determining a risk indicator for a medical complication event associated with a patient during a hospitalization in an intensive care unit and/or an intermediate care unit and/or a telemetry unit of a first clinical site, in particular following a surgery, the risk indicator determination unit configured to

> acquire, during the hospitalization, manually and/or automatically collected clinical data of the set of measurement-based clinical parameters associated with the patient, the data comprising at least a subset of the clinical parameters collected with a plurality of different respective sampling frequencies; and

> determine the risk indicator by using a prediction function generated as described above, in particular using the aforementioned prediction function generation unit, wherein the prediction function takes the clinical data as an input.

The prediction function generation unit and/or the risk indicator determination unit may be configured to additionally assess the performance of the prediction function by generating at least one performance metric by using, as the input of the prediction function, local assessment data comprising patient data originating from the first clinical site. For instance, the risk indicator determination unit may be configured to receive or retrieve the initially generated and initially assessed prediction function and additionally assess it using local assessment data as described.

In an example, means for generating and initially assessing the prediction function may be provided in the form of instructions stored in a memory of a prediction function generation unit implemented, for instance, as a general-purpose computer at a manufacturer site, the computer having access (e.g. via a network connection) to the external training and assessment data. Means for executing the prediction function (i.e. for determining the risk indicator) may be provided in the form of instructions stored in a memory of a risk indicator generation unit implemented, for instance, as a server computer at a deployment site, with individual terminals connected to the server computer provided at individual patient care locations of the deployment site for control of the risk indicator generation unit and/or display of the risk indicator and optionally further data. Alternatively, a risk indicator generation unit may be provided as part of each individual terminal. Means for additionally assessing and/or updating the prediction function may be provided within the prediction function generation unit and/or the risk indicator generation unit. As evident to the skilled person, the described system configurations are merely exemplary, and many alternative implementations are possible.

At least one of the one or more second clinical sites is distinct from the first clinical site. Distinct clinical sites are typically different hospitals, but may also include, for instance, different units within the same hospital. In broad terms, the first clinical site or sites is/are the provenance of the external training and assessment data used to (initially) prepare and assess the model and prediction function before deployment, while the second clinical site is typically a deployment site, where a prediction of the risk of medical complications is desired. Additional assessment is preferably performed at the deployment site.

Typically, assessment is followed - if one or several performance metrics are outside of the respective target range - by replacement and/or modification, such as retraining and/or fine-tuning, of the machine learning model.

At any stage, the machine learning model may additionally be validated using, for instance, standardized datasets. Here, validation refers to the process of assessing the performance of the prediction function by generating one or several performance metrics and determining whether said performance metrics fall within a respective target range. Alternatively or additionally, assessment and/or validation may be performed using a prediction function based on a dummy model (for instance, a random classifier that picks a classification based on the overall ratio/prevalence of the classes in a dataset) and/or using a reference clinical prediction model, where available (such as the established Bojar algorithm for identification/prediction of critical bleeding events).

The external training and/or the external assessment data may be or comprise historical data from a comparatively large cohort (referred to as external training cohort) of patients with known clinical histories and/or outcomes. The external training cohort may be composed of patients having undergone a specific treatment or intervention, such as a specific surgery, in particular a cardiothoracic surgery. The historical data preferably is or comprises monitoring data of the patients during hospitalization at the second clinical site or sites following said treatment or intervention. The known clinical histories and/or outcomes may, in particular, comprise an indication whether and when the medical complication event occurred during the hospitalization.

The external assessment data is preferably of the same type and structure, but from a distinct cohort of patients (referred to as external assessment cohort) than the external training data such as to enable independent assessment of the performance of the model and prediction function. A dataset size of the external assessment data (corresponding to a cohort size of the external assessment cohort) may be smaller than a dataset size of the external training data (corresponding to a cohort size of the external training cohort).

The local assessment data may be or comprise historical data from a local assessment cohort composed of patients hospitalized at the first clinical site. The local assessment data is preferably of the same general type and structure as the external training and assessment data and preferably includes at least mostly the same clinical parameters; however, it may differ from the external training and/or assessment data in the specific subset of clinical parameters included (e.g. some clinical parameters included in the external training and/or assessment data may be omitted from the local assessment data) and/or in further aspects such as sampling frequencies/sampling intervals and/or the equipment and/or methods used for obtaining certain clinical parameters. A dataset size of the external assessment data (corresponding to a cohort size of the local assessment cohort) may be smaller than the dataset size of the external training data.

The prediction function may be updated by training the machine learning model using local training data comprising patient data originating from the first clinical site when a pre-defined condition is satisfied and/or at pre-defined times and/or when a pre-defined event occurs. Updating the prediction function in this way further enables or contributes to reduction or avoidance of negative impacts of any dataset shift of the aforementioned types on model and prediction function performance.

Updating the prediction function by training the machine learning model using local training data is preferably performed by fine-tuning the machine learning model using the local training data. Fine-tuning is understood to additionally train the initially generated machine learning model (also referred to as pre-trained model or foundation model) using additional data, in particular such as to update at least a subset of the plurality of weights of the foundation model, where the foundation model is a neural network. Typically, at least a subset of the plurality of layers of the neural network are unfrozen during fine-tuning, which means that the weights associated with said subset of layers are allowed to vary during training with the additional data, while weights associated with any remaining layers remain fixed.

For instance, the prediction function may be updated in the above-described manner before the prediction function is used, in particular for the first time after deployment, for determining the risk indicator and/or if the at least one performance metric generated when additionally assessing the performance of the prediction function using local assessment data is outside of a target range.

The local training data, like the local assessment data, may be or comprise historical data from a local training cohort composed of patients hospitalized at the first clinical site. The local training data, like the local assessment data, is preferably of the same general type and structure as the external training and assessment data and preferably includes at least mostly the same clinical parameters; however, it may differ from the external training and/or assessment data as noted above with respect to the local assessment data. A dataset size of the local training data (corresponding to a cohort size of the local training cohort) may be smaller than the dataset size of the external training data.

The step of additionally assessing the performance of the prediction function using local assessment data and/or the step of updating the prediction function by training the machine learning model using local training data may be repeated after a specified time interval and/or in response to a specified event, in particular using additional local assessment and/or training data generated during the specified time interval. This aspect further enables or contributes to reduction or avoidance of negative impacts of any dataset shift of the aforementioned types on model and prediction function performance.

Specified events of the aforementioned type may include, for example, changes to infrastructure present and/or procedures followed at the first clinical site and/or to the composition of relevant patient cohorts and/or safety events.

The method for determining the risk indicator may comprise outputting the risk indicator for subsequent use in a prognostic and/or diagnostic and/or therapeutic assessment to be performed by medical personnel.

The risk indicator may in particular be a risk indicator for a medical complication event associated with the patient during a hospitalization following surgery. Alternatively or additionally, the hospitalization may be associated, for instance, with a pharmacotherapy, chemotherapy, monitoring of a medical condition, and/or other clinical situations. The surgery may be a cardiothoracic surgery. The medical complication event may be or comprise a renal injury event and/or a bleeding event requiring surgical revision and/or any other complication event expectable in the context of the patient's hospitalization. For instance, renal injury events and bleeding events requiring surgical revision are critical complication events commonly associated with cardiothoracic surgery. By determining a risk indicator of the specified type for such events, it is possible to provide medical personnel with additional information to aid in decision making aimed at improving patient outcomes.

Determining the risk indicator may be considered as a (binary) classification problem pertaining to the classes "medical complication event occurs" and "medical complication event does not occur". The machine learning model acts as a classifier in that it must determine, for the data associated with a given patient, which of said classes the data is most likely associated with. The risk indicator may be provided simply as an indication of the most likely class and/or may comprise a numerical value, such as a score relating to a likelihood that the currently assessed data is associated with one of the classes.

The at least on performance metric generated when initially and/or additionally assessing the performance of the prediction function may, for instance, comprise one or more metrics selected from the group comprising sensitivity, specificity, positive predictive value, negative predictive value, area under the receiver operating characteristic, and F1 score.

The at least one performance metric generated when initially and/or additionally assessing the performance of the prediction function may comprise a final-point metric and/or a reverse-interval metric and/or a forward-interval metric and/or a composite performance index; these metric types refer to the way a statistic metric (such as the above-mentioned sensitivity, specificity, positive predictive value, negative predictive value, area under the receiver operating characteristic, and F1 score) is evaluated and may therefore be referred to as an evaluation method.

Here, a final-point metric is defined as a metric calculated for a last data point or last time window (e.g. last minute) of data points of a time series. Such a metric evaluates model performance closest to the predicted medical complication event, when prediction accuracy is of particular importance.

A reverse-interval metric is defined as a metric obtained by grouping a time series into intervals leading up to the predicted medical complication event, providing a time series up to a specific point before said event (such as, e.g., 1 h or 6 h before the event). Such a metric evaluates model performance for earlier time points.

A forward-interval metric is defined as a metric calculated over expanding time intervals moving forward from a starting point, e.g. beginning with data from the first hour (or other time window) of observations and progressively adding additional hours. Such a metric provides a cumulative view of model performance over time.

A composite performance index integrates multiple performance indicator or performance metrics into a single, scalar value. Such a performance index provides a simple, summary view of aspects of the performance of the model.

For example, for an evaluation method x (such as last-minute), a corresponding composite performance $PI_x$ may be calculated as

$$PI_x = \frac{\sum_{m \in M} w_m \cdot m_x}{\sum_{m \in M} w_m} \qquad \text{(equation 1)},$$

wherein $m_x$ are the statistical metrics (such as the above-mentioned sensitivity, specificity, positive predictive value, negative predictive value, area under the receiver operating characteristic, and F1 score) calculated using evaluation method x, and $w_m$ (with $m \in M$) are weights of the individual statistical metrics.

For time-resolved evaluation methods y (such as forward-interval or reverse-interval), time averaging also has to be performed. Correspondingly, a corresponding composite performance index $PI_y$ may be calculated as

$$PI_y = \frac{\sum_{t \in T} w_t \cdot PI_{y,t}}{\sum_{t \in T} w_t} \qquad \text{(equation 2)},$$

wherein $PI_{y,t}$ are the values of an individual performance index at different time points (calculated as above), and $w_t$ (with $t \in T$) are weights of the individual time points.

A total composite performance index *Total PI* may be obtained by weighted averaging of the performance indices for individual evaluation methods, i.e. by calculating

$$Total\ PI = \frac{\sum_{z \in Z} w_z \cdot PI_z}{\sum_{z \in Z} w_z} \qquad \text{(equation 3)},$$

wherein $PI_z$ (with $z \in Z$) are the performance indices for individual evaluation methods, and $w_z$ are respective weights.

The local and/or external training and/or assessment data may be pre-filtered and/or evaluated and/or pre-processed before use in model training and/or assessment, in particular as described in the following.

Pre-filtering of the local and/or external training and/or assessment data may include selecting patient data base on specific criteria, in particular selecting data only from patients having undergone a pre-specified procedure, such as a cardiothoracic surgery, and having subsequently been admitted into an intensive care unit and/or an intermediate care unit and/or a telemetry unit of the one or more second clinical sites and/or selecting data from only the first (of potentially several) relevant procedure of the pre-specified type and/or selecting only data from patients with "complete" datasets (i.e. comprising all of a specified set of clinical and/or demographic parameters).

Evaluation of the local and/or external training and/or assessment data may include assessment of the availability and comparability of specific clinical parameters within the data. Comparability may be assessed using metrics such as sampling interval/sampling frequency and/or statistical measures such as mean values, standard deviations and the like, in particular by determining deviations (e.g. in percent) of such metrics between different datasets. Evaluation of this kind may permit decisions on whether a dataset is generally suitable for use with the proposed method, systems and products.

Pre-processing of the local and/or external training and/or assessment data may include, for instance, normalizing and/or rescaling and/or converting data associated with certain clinical parameters (e.g. such as to be provided in pre-specified units) and/or filling in missing data points according to pre-specified rules (for instance, setting missing data points to 0 or to a value of a preceding data point).

The local and/or external training and/or assessment data may comprise, for each associated patient, an event indicator indicating whether or not the medical complication event occurred within a preset time window and, if the medical complication event occurred, a time of occurrence of the medical complication event. During training, the event indicator may serve as a label, in particular for the purposes of supervised learning. During assessment and/or validation, the event indicator may serve as an indicator of the accuracy of the risk indicator. In these cases, in order to avoid label leakage, the event indicator should be excluded from the input of the prediction function during assessment and/or validation. The preset time period may also be referred to as a critical event horizon, as it specified the timeframe within which a critical event (pertinent medical complication event) must occur to be considered relevant for la-beling.

The local and/or external training data may further include or be processed such as to include, for each associated patient, a label time series comprising, for each of a plurality of time points of the data associated with the respective patient, a binary label;

  wherein, if the event indicator indicates that the medical event occurred within the preset time window, the binary label is set to a first value for each time point of the label time series starting at a time point defined as the beginning of a preset period of time before the time of occurrence of the medical complication event, and is set to a second value for each time point of the label time series preceding said time point;

  wherein, if the event indicator indicates that the medical event did not occur within the specified time window, the binary label is set to the second value for each time point of the label time series.

The first and second value may in particular correspond to Boolean values such as "TRUE" and "FALSE", respectively, or 1 and 0, respectively. The above-defined preset period of time before the time of occurrence of the medical complication event is also referred to as the prediction horizon and corresponds to the period before the complication event during which predictions are made. The label time series may also be referred to as a target output time series. In the context of training the machine learning model, such a target output time series acts as a classification guide or template for the patient data to be processed when determining the risk indicator.

The machine learning model may be or comprise a continuous-output classifier with an output value within an output value range.

Determining the risk indicator may comprise scaling or calibrating the risk indicator and/or the output value range for interpretability and/or such as to provide specific statistical information. For instance, the risk indicator and/or the output value range may be configured in such a way that a value of the risk indicator corresponds to an empirical probability of occurrence of the medical complication event. The empirical probability and/or a conversion map (such as a calibration curve) to calculate the empirical probability may be provided as a model output of the machine learning model, i. e. may be learned based on the local and/or external training data.

Determining the risk indicator may comprise setting a classification threshold within the output value range, wherein the classification threshold is set such as to maximize a determined model performance metric, in particular an F1 score. Other model performance metrics, such as an $F\beta$ score with $\beta \neq 1$, may be used alternatively or additionally. The classification threshold relates to the above-described classification problem pertaining to the classes "medical complication event occurs" and "medical complication event does not occur". Setting the threshold such as to maximize a determined model performance metric ensures optimal classification performance. It is noted that the classification threshold corresponding to the maximized performance metric may vary throughout a patient's observation period.

If using the classification threshold as described, determining the risk indicator may further comprise rescaling the output value range such that the classification threshold corresponds to a preset value of the output value range, preferably a midpoint of the output value range. Such a rescaling may, in particular, ensure that the risk indicator is intuitively understandable for a user (medical personnel). For instance, in a scoring range from 0 to 100, a score of 50 is typically perceived as a "natural" dividing line between probable and improbable outcomes.

The method for generating a prediction function may further comprise generating a plurality of prediction functions based on respective machine learning models trained using respective datasets, which differ in at least one characteristic parameter of associated data, in particular a sampling frequency and/or sampling interval. Correspondingly, the method for determining the risk indicator may further comprise selecting the prediction function from a plurality of prediction functions based on respective machine learning models trained using respective datasets, which differ in at least one characteristic parameter of associated data, in particular a sampling frequency and/or sampling interval. By providing several models, it is possible to select a model most closely aligned with data collection procedures or equipment at the deployment site. The choice of an appropriate model may be based on characteristic parameters (such as sampling frequency/sampling interval) or on generation and comparison of model performance metrics, such as the metrics described in this document.

The prediction function may be configured to determine a plurality of risk indicators for respective medical complication events associated with the patient during the hospitalization. A plurality of prediction functions may be provided (in particular, generated, assessed, and used as described herein), each prediction function configured to determine a risk indicator for a respective one of a plurality of medical complication events associated with the patient during the hospitalization.

In the following, examples of useful clinical parameters are provided preceded by corresponding standardized LOINC (Logical Observation Identifiers Names and Codes) identifiers, where applicable. It is noted that the LOINC identifiers are useful for unambiguous identification, conversion, or mapping of available clinical parameters, but one or several of the following and/or similar and/or additional parameters may be used without reference to any standardized identifiers or classification.

The measurement-based clinical parameters may include vital variables, such as one or more of 8867-4 Heart rate, 59408-5 Oxygen saturation in Arterial blood by Pulse oximetry, 60985-9 Central venous pressure (CVP), 8310-5 Body temperature, 8478-0 Mean blood pressure, 8480-6 Systolic blood pressure, 8462-4 Diastolic blood pressure, 8414-5 Pulmonary artery Mean blood pressure, 8385-7 Pulmonary artery Diastolic blood pressure, 8440-0 Pulmonary artery Systolic blood pressure.

The measurement-based clinical parameters may include analytical results, in particular laboratory and/or blood-gas and/or point-of-care lab results, such as one or more of 718-7 Hemoglobin [Mass/volume] in Blood, 2019-8 Carbon dioxide [Partial pressure] in Arterial blood, 2703-7 Oxygen [Partial pressure] in Arterial blood, 33254-4 pH of Arterial blood adjusted to patient's actual temperature, 30242-2 Lactate [Mass/volume] in Arterial blood, 2711-0 Oxygen saturation in Venous blood, 14979-9 aPTT in Platelet poor plasma by Coagulation assay, 6301-6 INR in Platelet poor plasma by Coagulation assay, 777-3 Platelets [#/volume] in Blood by Automated count, 1996-8 Calcium [Moles/volume] in Blood, 26464-8 Leukocytes [#/volume] in Blood, 2708-6 Oxygen saturation in Arterial blood, 1960-4 Bicarbonate [Moles/volume] in Arterial blood, 1925-7 Base excess in Arterial blood by calculation, 14627-4 Bicarbonate [Moles/volume] in Venous blood, 4544-3 Hematocrit [Volume Fraction] of Blood by Automated count, 2324-2, Gamma glutamyl transferase [Enzymatic activity/volume] in Serum or Plasma, 2947-0 Sodium [Moles/volume] in Blood, 6298-4 Potassium [Moles/volume] in Blood, 2339-0 Glucose [Mass/volume] in Blood, 6299-2 Urea nitrogen [Mass/volume] in Blood, 2021-4 Carbon dioxide [Partial pressure] in Venous blood, 1927-3 Base excess in Venous blood by calculation, 2705-2 Oxygen [Partial pressure] in Venous blood, 2746-6 pH of Venous blood, 2716-9 Fractional oxyhemoglobin in Venous blood, 30241-4 Lactate [Mass/volume] in Venous blood, 785-6 MCH [Entitic mass] by Automated count, 789-8 Erythrocytes [#/volume] in Blood by Automated count, 786-4 MCHC [Mass/volume] by Automated count, 787-2 MCV [Entitic volume] by Automated count, 788-0 Erythrocyte distribution width [Ratio] by Automated count, 32623-1 Platelet mean volume [Entitic volume] in Blood by Automated count, 58413-6 Nucleated erythrocytes/100 leukocytes [Ratio] in Blood by Automated count, 771-6 Nucleated erythrocytes [#/volume] in Blood by Automated count, 2157-6 Creatine kinase [Enzymatic activity/volume] in Serum or Plasma, 2160-0 Creatinine [Mass/volume] in Serum or Plasma, 32673-6 Creatine kinase.MB [Enzymatic activity/volume] in Serum or Plasma, 2532-0 Lactate dehydrogenase [Enzymatic activity/volume] in Serum or Plasma, 1988-5 C reactive protein [Mass/volume] in Serum or Plasma.

The measurement-based clinical parameters may include volume status variables, such as one or more of 81661-1 Blood loss, 9187-6 Urine output.

The prediction function may additionally take immutable and/or quasi-immutable parameters associated with the patient, for example age and/or birth date and/or gender and/or duration of the surgery and/or body weight, as an input. Correspondingly, the local and/or external training data and the local and/or external assessment data may additionally comprise data of said immutable and/or quasi-immutable parameters associated with each of the corresponding patients.

The Figures described in the following illustrate exemplary embodiments, principles, and teachings of the presently disclosed subject matter. In the Figures:

FIG. 1 shows, schematically, a medical data processing system,

FIG. 2 shows, schematically, a prediction function generation unit,

FIG. 3 shows, schematically, a risk indicator determination unit and a terminal,

FIG. 4 shows a flow diagram of a method for generating a prediction function,

FIG. 5 shows a flow diagram of a method for determining a risk indicator,

FIG. 6 shows, schematically, connections between different patient cohorts, data, and models.

Recurring and similar features in the Figures are provided with identical or similar alphanumerical reference signs. These may be partially omitted when the corresponding features are also shown in and described with reference to other Figures and/or not referenced in the context of a Figure description.

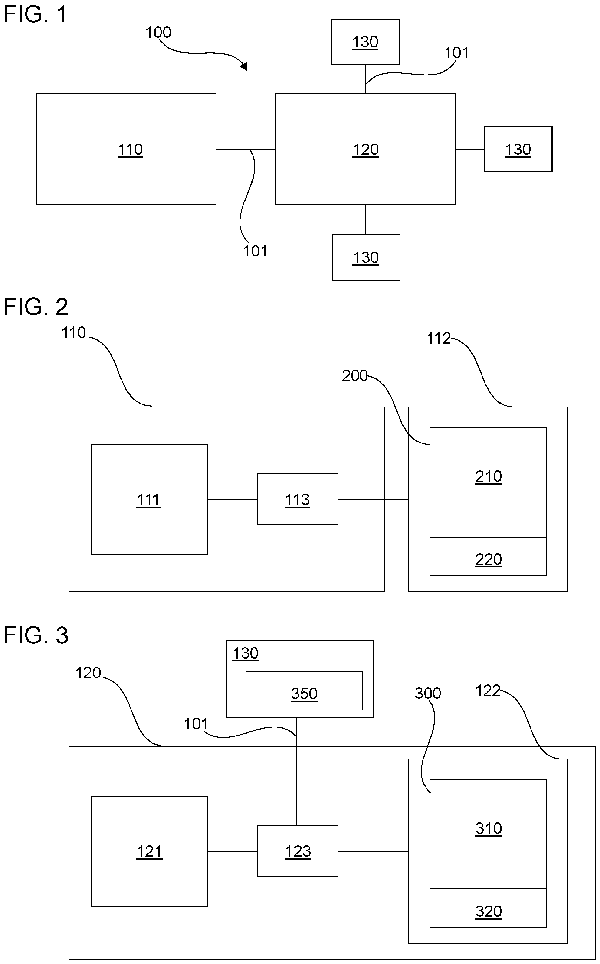

The medical data processing system 100 illustrated in FIG. 1 comprises a prediction function generation unit 110 (first medical data processing device) configured to generate a prediction function and a risk indicator determination unit 120 (second medical data processing device) connected to the prediction function generation unit 110 via a network connection 101. The risk indicator determination unit 120 is deployed at a deployment site, the deployment site being a first clinical site, such as a first hospital. The network connection 101 is merely an example; communication between the prediction function generation unit 110 and the risk indicator determination unit 120 may be provided by any of a variety of means, which further include, for instance, exchange of a storage device and/or direct integration of the prediction function generation unit 110 and the risk indicator determination unit 120 within the same computer system. The prediction function generation unit 110 is typically, though not necessarily, deployed at a manufacturer or system integrator site.

The risk indicator determination unit 120 is configured to receive (for instance, from the prediction function generation unit 110 via network connection 101 and/or a storage device and/or other means), one or several generated prediction functions and to determine, using the received prediction function, a risk indicator for a medical complication event. The risk indicator determination unit 120 is connected (via network connections 101) to a plurality of terminals 130 (third data processing devices) provided at individual patient care locations of the deployment site for control of the risk indicator generation unit 120 and display of the risk indicator as well as (optionally) further data, such as additional patient-related data. The patient care locations are part of an intensive care unit, an intermediate care unit, a telemetry unit or the like of a first clinical site, wherein patients are hospitalized following a surgery or other treatment or intervention, in particular a cardiothoracic surgery. A risk of future occurrence the medical complication event is associated with the treatment or intervention; the risk indicator is calculated to provide an indication or estimate of such a risk as a basis for further decision making by healthcare personnel.

The presently described system architecture as shown in FIG. 1-3 is exemplary. Here, each of the prediction function generation unit 110, the risk indicator determination unit 120, and the terminals 130 are general-purpose computers configured as servers or workstations, respectively, and communicate via network connections. In other examples, a risk indicator generation unit 110 may be provided as part of each individual terminal 130. It will further be appreciated that implementations using, for instance, dedicated hardware and various types of local and/or distributed computing architectures are possible. The provided functionality as a whole or individual aspects thereof may be implemented in hardware. Preferably, the functionality of generating the prediction function and of determining the risk indicator is provided in the form of software comprising instructions which, when executed on the respective devices, in particular the function generation unit 110 and/or the risk indicator determination unit 120, cause the methods and functionalities described herein to be executed. In further examples, the first and/or second and/or third medical data processing device may be combined into a single medical data processing device. Where reference is made to a central processing unit (CPU), other processing means, such as a plurality of CPUs and/or one or several graphics processing units (GPU) and/or one or several tensor processing units (TPU), may be used alternatively or additionally.

As illustrated in FIG. 2, means for generating and initially assessing a performance of the prediction function are provided in the form of instructions stored in a memory 111 of prediction function generation unit 110 implemented, in the present example, as a general-purpose computer at the manufacturer site, the computer having access to external data 200 stored on an external storage device 112. The external data 200 comprises external training data 210 as well as external assessment data 220. A central processing unit (CPU) 113 is configured to execute the instructions to generate and initially assess the performance of the prediction function.

As illustrated in FIG. 3, means for executing the prediction function (i.e. for determining the risk indicator) are provided in the form of instructions stored in a memory 121 of risk indicator generation unit 120 implemented, in the present example, as a server computer at the deployment site, the computer having access to local data 300 stored on an internal storage device 122. The local data 300 comprises local assessment data 320 and, preferably, local training data 310. A central processing unit (CPU) 123 is configured to execute the instructions to execute the prediction function in order to additionally assess the prediction function and/or to determine the risk indicator associated with a hospitalized patient. For determining the risk indicator associated with the hospitalized patient, the risk indicator generation unit 120 further has access - via network connection 101 to terminal 130 - to manually and/or automatically collected clinical data (referred to as hospitalization data 350) of a set of measurement-based clinical parameters associated with the patient, the data comprising at least a subset of the clinical parameters collected with a plurality of different respective sampling frequencies. Collection of the hospitalization data 350 is continuously ongoing during hospitalization. Preferably, the risk indicator generation unit 120 is further configured to update the prediction function using the local training data 310.

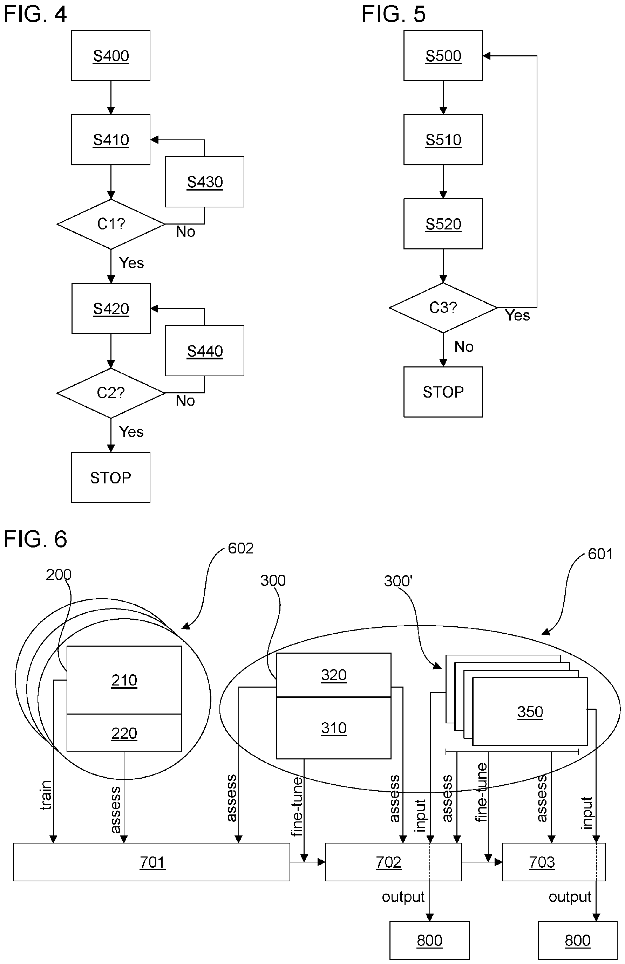

The medical data processing system 100 is configured to execute a method for generating the prediction function as illustrated in FIG. 4, the method comprising at least the steps:

Step S400: initially generating the prediction function by training a machine learning model using external training data 210 comprising patient data originating from one or more second clinical sites, the patient second exemplary use case, the medical complication event is a bleeding event For both exemplary use cases, all of the following parameters (preceded by corresponding standardized LOINC identifiers, where applicable), are used as inputs to generate the prediction function/determine the risk indicator:

- data comprising manually and/or automatically collected data of a set of measurement-based clinical parameters associated with a plurality of patients, the data comprising at least a subset of the clinical parameters collected with a plurality of different respective sampling frequencies;
- Step S410: initially assessing a performance of the prediction function by generating at least one performance metric by using, as the input of the prediction function, external assessment data 220 comprising patient data originating from the one or more second clinical sites; and
- Step S420: additionally assessing the performance of the prediction function by generating at least one performance metric by using, as the input of the prediction function, local assessment data 320 comprising patient data originating from the first clinical site.

In the medical data processing system 100 according to the above-described example, the prediction function generation unit 110 is configured to execute steps S400 and S410, whereas the risk indicator determination unit 120 is configured to execute step S420. Correspondingly, the risk indicator determination unit 120 is configured to receive or retrieve the initially generated and initially assessed prediction function and additionally assess it using local assessment data 320 as described.

The machine learning model generated in step S400 is preferably a deep learning neural network, in this example a transformer model. However, other types of models, such as a recurrent neural network (RNN), logistic regression, support vector machine, or random forest may alternatively be used.

Each of steps S410 and S420 is executed at least once. After completion of step S410, condition C1 may be evaluated. Condition C1 is satisfied if the at least one performance metric generated in step S410 falls within a target range (or each of several performance metrics falls within a respective target range). If condition C1 is not satisfied, the initially generated model may undergo further optimization (step S430), which may include, for instance, choice of a different model and/or further training and/or adjustment of hyperparameters and/or changes to the external training data (e.g. use of a larger or smaller dataset).

If condition C1 is satisfied, the initially generated model is considered sufficiently optimized. This model is also referred to as the foundation model. The foundation model is typically generated (i.e. steps S400, S410, and S430 are typically performed) at the manufacturer or system integrator site before deployment of the prediction function.

Step S420 is typically performed at the deployment site (e.g. after installation of corresponding hardware and/or software at the first clinical site), but could also be performed, for example, at the manufacturer or system integrator site using data from the deployment site. After completion of step S420, condition C2 is evaluated. Condition C2 is satisfied if the at least one performance metric generated in step S420 falls within a target range (or each of several performance metrics falls within a respective target range).

If condition C2 is not satisfied, the prediction function is, in a step S440, updated by fine-tuning (see above) the foundation model using the local training data 310 comprising patient data originating from the first clinical site.

It is noted that in addition or alternatively to condition C2 not being satisfied, updating of the prediction function (step S440) may occur when a different pre-defined condition is satisfied and/or at pre-defined times and/or when a s event occurs.

Similarly, the step of additionally assessing the performance of the prediction function using local assessment data (step S420) may be repeated after a specified time interval and/or in response to a pre-defined event, in particular using additional local assessment and/or training data generated during the specified time interval. Pre-defined events in the aforementioned scenarios may include, for example, changes to infrastructure present and/or procedures followed at the first clinical site and/or to the composition of relevant patient cohorts and/or safety events.

If condition C2 is satisfied, the prediction function model is considered sufficiently optimized and ready for deployment in determining the risk indicator associated with patients (here indicated as a STOP of the method for generating the prediction function).

The risk indicator determination unit 120 is configured to execute a method for determining the risk indicator as illustrated in FIG. 5, the method comprising:

- Step S500: acquiring, during the hospitalization, manually and/or automatically collected clinical data of the set of measurement-based clinical parameters associated with the patient, the data comprising at least a subset of the clinical parameters collected with a plurality of different respective sampling frequencies;
- Step S510: determining the risk indicator by using a prediction function generated using the method for generating a prediction function described above, wherein the prediction function takes the clinical data as an input; and
- Step S520: outputting the risk indicator for subsequent use in a prognostic and/or diagnostic and/or therapeutic assessment to be performed by medical personnel (for instance on a display of terminal 130 and/or in a memory of terminal 130 and/or a storage device).

After completion of Step S510 and S520, condition C3 is evaluated. Condition C3 is a continuation condition that is satisfied while monitoring of the patient is ongoing and a mode of repeatedly determining the risk indicator is active. If condition C3 is satisfied, steps S510 and S520 are repeated; otherwise, the procedure is terminated (indicated as STOP). Alternatively, the system may be configured, for instance, such that a subsequent update of the risk indicator is provided whenever such an update is requested by a user and/or a part of the system.

Where, in the method steps described above, performance metrics are calculated, typically a set of final-point metrics and reverse-interval metrics and forward-interval metrics and composite performance indices (as described further above) is calculated for each of the statistic metrics from the group comprising sensitivity, specificity, positive predictive value, negative predictive value, area under the receiver operating characteristic, and F1 score.

The local training data 310, the local assessment data 320, the external training data 210, and the external assessment data 220 comprise, for each associated patient, an event indicator indicating whether or not the medical complication event occurred within a preset time window and, if the medical complication event occurred, a time of occurrence of the medical complication event. In order to avoid label leakage, the event indicator is excluded from the input of the prediction function during assessment/validation.

The local training data 310, the local assessment data 320, the external training data 210, and the external assessment data 220 are further, prior to use, processed such as to include, for each associated patient, a label time series as defined and described above serving as a classification guide.

The machine learning model is a continuous-output classifier with an output value within an output value range, wherein determining the risk indicator comprises setting a classification threshold within the output value range, wherein the classification threshold is set such as to maximize an F1 score. Different performance metrics (in place of the F1 score) and/or different output calibration methods (in place of the thresholding) may however be used, for example as described above (e.g. by scaling or calibrating the risk indicator and/or the output value range for interpretability and/or such as to provide specific statistical information, such as an empirical probability of occurrence of the medical complication event). Before outputting the risk indicator (step S520), rescaling is further performed such that the classification threshold corresponds to a preset value of the output value range, preferably a midpoint of the output value range, in order to ensure that the risk indicator is intuitively understandable for a user.

FIG. 6 provides an exemplary overview of how the machine learning model underlying the prediction function may be generated and updated over time in relation to the clinical sites and corresponding patient cohorts/patient data.

In the illustrated example, the external data 200 originate from multiple second clinical sites 602, such as multiple hospitals or multiple units of one or multiple hospitals (in other examples, the external data 200 may originate from a single clinical site, such as a single hospital or a single unit of a hospital). The local data 300 originate from a first clinical site 601.

Each of the external data 200 and the local data 300 comprise electronic health record (EHR) data containing patient data from a respective patient cohort, including immutable and/or quasi-immutable parameters associated with the respective patient as well as manually and/or automatically collected data of a set of measurement-based clinical parameters associated with the respective patients (longitudinal or time series data), said data comprising at least a subset of the clinical parameters collected with a plurality of different respective sampling frequencies.

Each of the external data 200 and the local data 300 may be pre-filtered and/or evaluated and/or pre-processed as described further above.

Each of the external data 200 and the local data 300 are split, without overlap, into a training dataset (external training data 210, local training data 310) and an assessment dataset (external assessment data 220, local assessment data 320).

The external training data 210 is used to initially generate the prediction function by training the foundation model 701. Subsequently, the foundation model 701 is initially assessed using the external assessment data 220, i.e. performance of the corresponding prediction function is assessed by generating one or several performance metrics, followed by determining whether said performance metrics fall within a respective target range. Additionally, the foundation model 701 may be validated using, for instance, standardized datasets ("external validation", not shown).

The foundation model 701 is additionally assessed/validated ("local validation") using the local assessment data 320. If required (see e.g. description of FIG. 4), fine-tuning is performed using the local training data 310 to obtain the updated model 702, whose performance may again be validated using the local assessment data 320.

During hospitalization of patients at the first clinical site 601, further local data 300' is generated in the form of hospitalization data 350 for each relevant patient (EHR data associated with hospitalized patients, shown here as a plurality of hospitalization datasets 350 for the respective patients).

Hospitalization data 350 of a given patient is used as an input of the prediction function based on the updated model 702 (if no update was required based on the local validation, the foundation model 701 may instead be used) to determine, as an output, the corresponding risk indicator 800.

The further local data 300' generated and accumulated over time may further be used to (periodically or triggered by certain events) perform further local validation of the updated model 702. If one or more performance metrics generated during this further local validation no longer fall within respective target ranges, additional fine-tuning may be performed using the further local data 300' to obtain a further updated model 703, followed by further local validation of the further updated model 703 using the further local data 300'. For the further update and further local validation, the further local data 300' may again be split into local training and assessment datasets. The external assessment data 220 and/or the local assessment data 320 may additionally be used in the further validation of the updated model 702 and/or the further updated model 703 and/or subsequently further updated models.

The above-described procedure may be performed and repeated (periodically or triggered by certain events as described elsewhere in this document) to keep the deployed model up to date and validated, thus accounting for any dataset shift that may occur.

In the following, certain considerations related to two specific exemplary use cases of the disclosed subject matter are outlined. In both cases, the risk indicator concerns a medical complication event associated with the patient during a hospitalization following cardiothoracic surgery.

In the first exemplary use case, the medical complication event is a renal injury event. Such events may be defined as involving severe postoperative acute kidney injury (AKI), classified as either KDIGO stage 2 or 3 based on the KDIGO (Kidney Disease: Improving Global Outcomes) AKI guidelines.

In the second exemplary use case, the medical complication event is a bleeding event requiring surgical revision. Such events may be defined by specifying the ICD-10 codes of corresponding revision surgeries, specifically codes I31.2 Hemopericardium, not elsewhere classified, I31.3 Pericardial effusion (non-inflammatory), I31.9 Disease of the pericardium, unspecified, J94.2 Hematothorax, T81.0 Hemorrhage and hematoma as a complication of surgery, not elsewhere classified, T81.1 Shock during or resulting from surgery, not elsewhere classified, T82.8 Other specified complications due to prostheses, implants or grafts in the heart and vessels.

For both exemplary use cases, all of the following parameters (preceded by corresponding standardized LOINC identifiers, where applicable), are used as inputs to generate the prediction function/determine the risk indicator:

Vital variables: 8867-4 Heart rate, 59408-5 Oxygen saturation in Arterial blood by Pulse oximetry, 60985-9 Central venous pressure (CVP), 8310-5 Body temperature, 8478-0 Mean blood pressure, 8480-6 Systolic blood pressure, 8462-4 Diastolic blood pressure, 8414-5 Pulmonary artery Mean blood pressure, 8385-7 Pulmonary artery Diastolic blood pressure, 8440-0 Pulmonary artery Systolic blood pressure.

Analytical results: 718-7 Hemoglobin [Mass/volume] in Blood, 2019-8 Carbon dioxide [Partial pressure] in Arterial blood, 2703-7 Oxygen [Partial pressure] in Arterial blood, 33254-4 pH of Arterial blood adjusted to patient's actual temperature, 30242-2 Lactate [Mass/volume] in Arterial blood, 2711-0 Oxygen saturation in Venous blood, 14979-9 aPTT in Platelet poor plasma by Coagulation assay, 6301-6 INR in Platelet poor plasma by Coagulation assay, 777-3 Platelets [#/volume] in Blood by Automated count, 1996-8 Calcium [Moles/volume] in Blood, 26464-8 Leukocytes [#/volume] in Blood, 2708-6 Oxygen saturation in Arterial blood, 1960-4 Bicarbonate [Moles/volume] in Arterial blood, 1925-7 Base excess in Arterial blood by calculation, 14627-4 Bicarbonate [Moles/volume] in Venous blood, 4544-3 Hematocrit [Volume Fraction] of Blood by Automated count, 2324-2, Gamma glutamyl transferase [Enzymatic activity/volume] in Serum or Plasma, 2947-0 Sodium [Moles/volume] in Blood, 6298-4 Potassium [Moles/volume] in Blood, 2339-0 Glucose [Mass/volume] in Blood, 6299-2 Urea nitrogen [Mass/volume] in Blood, 2021-4 Carbon dioxide [Partial pressure] in Venous blood, 1927-3 Base excess in Venous blood by calculation, 2705-2 Oxygen [Partial pressure] in Venous blood, 2746-6 pH of Venous blood, 2716-9 Fractional oxyhemoglobin in Venous blood, 30241-4 Lactate [Mass/volume] in Venous blood, 785-6 MCH [Entitic mass] by Automated count, 789-8 Erythrocytes [#/volume] in Blood by Automated count, 786-4 MCHC [Mass/volume] by Automated count, 787-2 MCV [Entitic volume] by Automated count, 788-0 Erythrocyte distribution width [Ratio] by Automated count, 32623-1 Platelet mean volume [Entitic volume] in Blood by Automated count, 58413-6 Nucleated erythrocytes/100 leukocytes [Ratio] in Blood by Automated count, 771-6 Nucleated erythrocytes [#/volume] in Blood by Automated count, 2157-6 Creatine kinase [Enzymatic activity/volume] in Serum or Plasma, 2160-0 Creatinine [Mass/volume] in Serum or Plasma, 32673-6 Creatine kinase.MB [Enzymatic activity/volume] in Serum or Plasma, 2532-0 Lactate dehydrogenase [Enzymatic activity/volume] in Serum or Plasma, 1988-5 C reactive protein [Mass/volume] in Serum or Plasma.

Volume status variables: 81661-1 Blood loss, 9187-6 Urine output.

Immutable/quasi-immutable parameters: age, gender.

For the first exemplary use case (renal injury events), the following parameters are additionally used: duration of the cardiothoracic surgery and 29463-7 Body weight.

List of reference signs

| | |
|---|---|
| 100 | medical data processing system, |
| 101 | network connection, |
| 110 | prediction function generation unit, |
| 111 | memory, |
| 112 | external storage device, |
| 113 | CPU, |
| 120 | risk indicator determination unit, |
| 121 | memory, |
| 122 | internal storage device, |
| 123 | CPU, |
| 130 | terminal, |
| 200 | external data, |
| 210 | external training data, |
| 220 | external assessment data, |
| 300 | local data, |
| 300' | further local data, |
| 310 | local training data, |
| 320 | local assessment data, |
| 350 | hospitalization data, |
| 601 | first clinical site, |
| 602 | second clinical site, |
| 701 | foundation model, |
| 702 | updated model, |
| 703 | further updated model, |
| 800 | risk indicator, |
| S400-S520 | method steps as described, |
| C1-C3 | flow control conditions as described. |

**Claims**

1. Medical data processing device (100, 110, 120), configured to

   receive or retrieve a pre-generated prediction function, the prediction function configured to determine a risk indicator (800) for a medical complication event associated with a patient during a hospitalization in an intensive care unit and/or an intermediate care unit and/or a telemetry unit of a first clinical site (601), in particular following a surgery, the pre-generated prediction function generated by training a machine learning model (701) using external training data (210) comprising patient data originating from one or more second clinical sites (602), the patient data comprising manually and/or automatically collected data of a set of measurement-based clinical parameters associated with a plurality of patients, the data comprising at least a subset of the clinical parameters collected with a plurality of different respective sampling frequencies; and
   assess (S420) the performance of the prediction function (S420) by generating at least one performance metric by using, as the input of the prediction function, local assessment data (320) comprising patient data originating from the first clinical site (601).

2. Medical data processing device (100, 110, 120) according to claim 1, further configured to

   acquire (S500), during the hospitalization, manually and/or automatically collected clinical data (350) of the set of measurement-based clinical parameters associated with the patient, the data comprising at least a subset of the clinical parameters collected with a plurality of different respective sampling frequencies;
   determine (S510) the risk indicator (800) by using the prediction function, wherein the prediction function takes the clinical data (350) as an input.

3. Method for generating a prediction function, the prediction function configured to determine a risk indicator (800) for a medical complication event associated with a patient during a hospitalization in an intensive care unit and/or an intermediate care unit and/or a telemetry unit of a first clinical site (601), in particular following a surgery, the method comprising

   initially generating (S400) the prediction function by training a machine learning model (701) using external training data (210) comprising patient data originating from one or more second clinical sites (602), the patient data comprising manually and/or automatically collected data of a set of measurement-based clinical parameters associated with a plurality of patients, the data comprising at least a subset of the clinical parameters collected with a plurality of different respective sampling frequencies; initially assessing (S410) a performance of the prediction function by generating at least one performance metric by using, as the input of the prediction function, external assessment data (220) comprising patient data originating from the one or more second clinical sites (602); additionally assessing (S420) the performance of the prediction function (S420) by generating at least one performance metric by using, as the input of the prediction function, local assessment data (320) comprising patient data originating from the first clinical site (601).

4. Method for determining a risk indicator (800) for a medical complication event associated with a patient during a hospitalization in an intensive care unit and/or an intermediate care unit and/or a telemetry unit of a first clinical site (601), in particular following a surgery, the method comprising

acquiring (S500), during the hospitalization, manually and/or automatically collected clinical data (350) of the set of measurement-based clinical parameters associated with the patient, the data comprising at least a subset of the clinical parameters collected with a plurality of different respective sampling frequencies; determining (S510) the risk indicator (800) by using a prediction function generated using the method according to claim 3, wherein the prediction function takes the clinical data (350) as an input.

5. Method according to any one of claims 3 and 4, wherein, before using the prediction function for determining the risk indicator (800) and/or if the at least one performance metric generated when additionally assessing the performance of the prediction function using local assessment data (320) is outside of a target range, the prediction function is updated (S440) by training, in particular fine-tuning, the machine learning model (701) using local training data (310) comprising patient data originating from the first clinical site (601).

6. Method according to any one of claims 3 through 5, wherein the step of additionally assessing the performance of the prediction function using local assessment data (320) and/or the step of updating the prediction function by training the machine learning model (701) using local training data (310) is repeated after a specified time interval and/or in response to a specified event, in particular using additional local assessment and/or training data generated during the specified time interval and/or since the prediction function was last updated.

7. Method according to any one of claims 4 through 6, comprising outputting the risk indicator (800) for subsequent use in a prognostic and/or diagnostic and/or therapeutic assessment to be performed by a medical provider.

8. Method according to any one of claims 3 through 7, wherein the surgery is a cardiothoracic surgery and/or the medical complication event is or comprises a renal injury event and/or a bleeding event requiring surgical revision.

9. Method according to any one of claims 3 through 8, wherein the machine learning model (701) is or comprises a supervised model, in particular a deep learning model, preferably a transformer model.

10. Method according to any one of claims 3 through 9, wherein the at least one performance metric generated when initially and/or additionally assessing the performance of the prediction function comprises a final-point metric and/or a reverse-interval metric and/or a forward-interval metric and/or a composite performance index.

11. Method according to any one of claims 3 through 10,

wherein the local and/or external training and/or assessment data comprise, for each associated patient, an event indicator indicating whether or not the medical complication event occurred within a preset time window and, if the medical complication event occurred, a time of occurrence of the medical complication event; wherein the local and/or external training data (210) includes or is processed such as to include, for each associated patient, a label time series comprising, for each of a plurality of time points of the data associated with the respective patient, a binary label; wherein, if the event indicator indicates that the medical event occurred within the preset time window, the binary label is set to a first value for each time point of the label time series starting at a time point defined as the beginning of a preset period of time before the time of occurrence of the medical complication event, and is set to a second value for each time point of the label time series preceding said time point; wherein, if the event indicator indicates that the medical event did not occur within the specified time window, the binary label is set to the second value for each time point of the label time series.

12. Method according to any one of claims 3 through 11, wherein the machine learning model (701) is or comprises a continuous-output classifier with an output value within an output value range, wherein determining the risk indicator (800) comprises setting a classification threshold within the output value range, wherein the classification threshold is set such as to maximize a determined model performance metric, in particular an F1 score.

13. Method according to any one of claims 3 through 12, further comprising selecting the prediction function from a plurality of prediction functions based on respective machine learning models (701) trained using respective datasets, which differ in at least one characteristic parameter of associated data, in particular a sampling frequency and/or sampling interval.

14. Method according to any one of claims 3 through 13,

wherein the measurement-based clinical parameters include vital variables, such as heart rate, body temperature, blood pressure and the like, and/or analytical results, in particular laboratory and/or blood-gas and/or point-of-care lab results, such as hemoglobin concentration, platelet count, creatinine concentration and the like, and/or volume status variables, such as blood loss, urine output and the like,
and/or
wherein the prediction function additionally takes immutable and/or quasi-immutable parameters associated with the patient, for example age and/or birth date and/or gender and/or duration of the surgery and/or body weight, as an input, and wherein the local and/or external training data (210) and the local and/or external assessment data (220) additionally comprise data of said immutable and/or quasi-immutable parameters associated with each of the corresponding patients.

15. Data processing system (100, 110, 120) comprising means for executing the method according to any one of claims 3 through 14.

16. Computer program product comprising instructions which, when executed on a data processing device (100, 110, 120), cause the data processing device to execute the method according to any one of claims 3 through 14.

FIG. 1

FIG. 2

FIG. 3

# FIG. 4

# FIG. 5

# FIG. 6

Europäisches
Patentamt

European
Patent Office

Office européen
des brevets

## EUROPEAN SEARCH REPORT

**Application Number**

EP 24 18 6862

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/212857 A1 (OUYANG DAVID [US] ET AL) 27 June 2024 (2024-06-27) <br> * paragraphs 12, 11, 42, 140-143, 37, 68, 127, 129, 70, 124, 99, 156; <br> figures 1, 10A, 10B, 12; <br> table 8; * | 1-16 | INV. <br> G16H50/20 <br> G16H50/30 <br> G16H50/70 |

-----

**TECHNICAL FIELDS SEARCHED (IPC)**

G16H

The present search report has been drawn up for all claims

| Place of search | Date of completion of the search | Examiner |
|---|---|---|
| Munich | 27 May 2025 | Wittke, Claudia |

EPO FORM 1503 03.82 (P04C01)

## ANNEX TO THE EUROPEAN SEARCH REPORT
## ON EUROPEAN PATENT APPLICATION NO.

EP 24 18 6862

This annex lists the patent family members relating to the patent documents cited in the above-mentioned European search report.
The members are as contained in the European Patent Office EDP file on
The European Patent Office is in no way liable for these particulars which are merely given for the purpose of information.

27-05-2025

| Patent document cited in search report | Publication date | Patent family member(s) | Publication date |
|---|---|---|---|
| US 2024212857 A1 | 27-06-2024 | US 2024212857 A1<br>WO 2022226227 A1 | 27-06-2024<br>27-10-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ZERVEAS et al.** A Transformer-based Framework for Multivariate Time Series Representation Learning. *arXiv:2010.02803v3*, 2020, https://arxiv.org/abs/2010.02803v3

- **KRITTANAWONG et al.** Artificial Intelligence in Precision Cardiovascular Medicine. *Journal of the American College of Cardiology*, 2017, vol. 69 (21), ISSN 0735-1097, 2657-2664